# EUROPEAN PATENT SPECIFICATION

(11) **EP 2 968 615 B1**
(45) Date of publication and mention of the grant of the patent: **03.07.2019**
(21) Application number: 14709701.8
(22) Date of filing: 13.03.2014
(51) Int. Cl.: A61K 49/00, A61K 31/137, A61K 31/4745, A61K 31/704, A61P 35/00

(54) **PARTICLES COMPRISING LUMINESCENT LANTHANIDE COMPLEXES**
PARTIKELN MIT LUMINESZENTEN LANTHANIDKOMPLEXEN
PARTICULES COMPRENANT DES COMPLEXES DE LANTHANIDE LUMINESCENT

(30) Priority: 14.03.2013 EP 13305291
(43) Date of publication of application: 20.01.2016
(73) Proprietor: Centre National de la Recherche Scientifique (C.N.R.S.), 75016 Paris (FR)
(72) Inventor: JAKAB TOTH, Eva, F-45160 Olivet (FR); PETOUD, Stéphane, F-75012 Paris (FR); LACERDA, Sara, London SE11 6NP (GB); BONNET, Célia, F-45000 Orléans (FR)
(74) Representative: Lavoix
(86) International application number: PCT/EP2014/055001
(87) International publication number: WO 2014/140203

(56) References cited:
- CÉLIA S. BONNET ET AL: "Hydrophobic chromophore cargo in micellar structures: a different strategy to sensitize lanthanide cations", CHEMICAL COMMUNICATIONS, vol. 46, no. 1, 1 January 2010 (2010-01-01), page 124, XP055071992, ISSN: 1359-7345, DOI: 10.1039/b918881a cited in the application
- C. GRANGE ET AL: "Combined Delivery and Magnetic Resonance Imaging of Neural Cell Adhesion Molecule-Targeted Doxorubicin-Containing Liposomes in Experimentally Induced Kaposi's Sarcoma", CANCER RESEARCH, vol. 70, no. 6, 15 March 2010 (2010-03-15) , pages 2180-2190, XP055072022, ISSN: 0008-5472, DOI: 10.1158/0008-5472.CAN-09-2821
- YANG CHEN ET AL: "Sensitized Luminescent Terbium Nanoparticles: Preparation and Time-Resolved Fluorescence Assay for DNA", ANALYTICAL CHEMISTRY, vol. 79, no. 3, 1 February 2007 (2007-02-01), pages 960-965, XP055072163, ISSN: 0003-2700, DOI: 10.1021/ac061477h
- LOÏC J. CHARBONNIÈRE ET AL: "Highly luminescent water-soluble lanthanide nanoparticles through surface coating sensitization", NEW JOURNAL OF CHEMISTRY, vol. 32, no. 6, 1 January 2008 (2008-01-01), page 1055, XP055072239, ISSN: 1144-0546, DOI: 10.1039/b719700d

## Description

The present invention relates to particles useful for drug delivery, particularly to luminescent particles.

Particles, notably nanoparticles such as liposomes and natural or synthetic lipoproteins, have been widely explored for drug delivery and biological imaging given their high payloads, improved detection sensitivity, long circulation times and the ease of integrating multiple properties. With particles, biological and biophysical barriers are more easily overcome to improve detection of pathophysiologic changes *in vivo.* Particle drug delivery also limits off-target toxicity associated with conventional medical therapies.

The control of the *in vivo* integrity of the particles and the monitoring of the release of the drug at the site of interest is one of the major fields where drug delivery can benefit from molecular imaging. This requires properly designed agents that allow *in vivo* visualization of the drug reaching a pathological region and the quantitative assessment of its release.

Organic fluorophores are notably used to test liposome content release: they are encapsulated in the liposomes above the self-quenching concentration and become detectable upon liposome rupture.

However, this method suffers from several disadvantages of organic fluorophores such as the difficulty of the detection of the fluorescence signal of the drug due to the interference of biological autofluorescence, photobleaching, change of emission wavelengths with the experimental conditions or reabsorption of the excitation light. Moreover, this is an indirect technique, since the detected signal is not directly related to the drug molecule.

Bonnet et al. (Chemical Communications, vol.46, no. 1, January 2010, pages 124-126) relates to micelles comprising luminescent lanthanide complexes which may comprise 2,3-naphthalimide in their core, but these micelles do not comprise any therapeutic agent.

Thus, there is a need in novel drug delivery technologies in order to increase efficacy or improve safety and patient compliance in the cure of highly social impact diseases, in particular cancer.

The present application aims at providing novel particles which allow the visualization of the drug release by optical imaging.

The present application particularly aims at providing novel liposomes and lipoproteins comprising a metal complex suitable for near-infrared (NIR) optical imaging.

The present invention relates to a particle, comprising:
- at least one luminescent metal complex of formula [Ln].[L], wherein:
   - Ln is a lanthanide ion,
   - L is an organic ligand comprising a hydrophilic unit with at least 6 chelating groups chosen from the group consisting of hydroxyl, carboxyl, optionally in the carboxylate form, carbonyl, ether, phosphonate, phosphinate, thiolate, amino and imino functions, and
- at least one drug comprising a conjugated aromatic system, wherein said drug is not covalently bonded to the metal complex, and wherein the drug is an anticancer agent,
said particle having a size comprised from 3 nm to 2 000 nm, and wherein the distribution of the metal complex and the drug within the particle is such that the mean distance between a drug molecule and a metal complex is less than 15 nm.

Among the constituents of the particles according to the present invention, at least one luminescent metal complex and at least one drug comprising a conjugated aromatic system have to be present.

Due to their unique electronic structures, lanthanides are well suited for the design of optical probes. Indeed, they possess advantageous optical properties, while their similar chemical reactivities allow facile substitution of one lanthanide by another. Luminescent lanthanide ions provide complementary properties to the above-presented organic fluorophores, such as resistance to photobleaching, long luminescence lifetimes, absence of reabsorption and sharp emission bands in the visible and the NIR. In comparison to visible detection, NIR luminescence results in improved signal-to-noise ratios and detection sensitivity in biological media due to the low residual cell/tissue autofluorescence.

It is also possible to simultaneously incorporate more than one metal complex into multiple-lanthanide containing particles, thus enabling "multiplex emission".

The particles according to the invention may further incorporate Gd³⁺ complexes, which are widely used as agents for MRI detection.

The particles according to the invention may further incorporate other lanthanide complexes providing the possibility of Paramagnetic Chemical Exchange Saturation Transfer (PARACEST) MRI detection.

The use of luminescent lanthanide requires a chromophore (also called "antenna") that is capable of absorbing excitation energy and transferring it to the metal complex.

The present invention consists of using the drug molecules, incorporated into the particles, as an antenna for the lanthanide luminescence. The inventors have found that the drug according to the invention is capable of absorbing excitation energy and transferring it to the metal complex, thus sensitizing the lanthanide luminescence and enabling the detection of the particle by optical means.

Since the drug is not covalently bonded to the metal complex, the lanthanide luminescence can exclusively be observed when the drug and the metal complex are within the same intact particle. "Not covalently bonded" means that the drug and the metal complex are not chemically attached and that they are two distinct species.

On the contrary, the release of the drug from the particles, or the destruction of said particles, will lead to the loss of the luminescence signal thus allowing a direct follow-up of the drug release, by optical means.

In case of particles further incorporating Gd³⁺ complexes, the biodistribution of the particles may also be monitored by MRI detection. This might allow monitoring the appropriate delivery of the particles to the desired biological site before content release (detected by optical means) happens.

Without being bound to a particular theory, the inventors have observed that, in particles having a size according to the invention, it is sufficient to distribute the drug molecules and the metal complex in the particles so that the mean distance between a drug molecule and a metal complex is less than 15 nm, in order to enable the above-mentioned energy transfer and thus the particle detection.

This condition can be met, for example by homogeneously distributing the drug and/or the metal complex within the particles, or within at least a zone of the particle, such as the core or the shell in the case of a core/shell particle.

In order to enable a luminescence suitable for optical detection, the skilled person in the art will be able to select the nature of the particles considering the hydrophilic or hydrophobic behavior of the metal complex and/or drug molecule.

For example, in the case of a liposome, i.e. in a particle comprising an aqueous core surrounded by a phospholipid bilayer, the drug and/or the metal complex may be homogeneously distributed within the core or the bilayer, according to the hydrophilic or hydrophobic nature of said drug and/or metal complex.

For example, in the case of a lipoparticle, i.e. in a particle comprising a lipidic core surrounded by a phospholipid monolayer, the drug and/or the metal complex may be homogeneously distributed within the core or the monolayer, according to the hydrophilic or hydrophobic nature of said drug and/or metal complex.

In case of low luminescence, the skilled person in the art will also be able to adapt the amounts of metal complex and/or drug molecule loaded into the particles in order to promote the energy transfer and thus enhance the lanthanide luminescence.

The invention provides a novel strategy for the luminescence sensitization of lanthanide without covalent bonds between the chromophore (i.e. the drug) and the lanthanide (i.e. the metal complex), and provides an unambiguous tool for monitoring the integrity of the particles, via emission in the NIR region.

This is the first report of a NIR emitting particle based on lanthanide luminescence. The structure and properties of these particles allow the localization in sufficient proximity of sensitizing antennae and NIR emitting lanthanide without the need of multistep organic synthesis. Furthermore, compared to methods using organic fluorophores, the technique according to the present invention is a direct technique, since the detected signal is directly related to the drug molecule.

Through the integration of targeting agents on the surface of the particles, such as proteins, synthetically or genetically modified proteins, natural or synthetic peptides, polysaccharides, natural or synthetic oligonucleotides, aptamers or small synthetic vectors, said particles can be specifically targeted to cellular receptors, such as LDL receptors over-expressed in cancer cells.

The versatility of particles also allows the simultaneous incorporation of luminescent lanthanides, magnetic probes such as Gd³⁺ complex, and drugs acting as a chromophore antenna.

The present invention can benefit to clinical healthcare and to drug development in the preclinical phase.

The particles of the invention may be used for the treatment of cancer.

The particles of the invention may be used for imaging methodologies, particularly for monitoring the release of the drug at its site of interest.

Several further aspects of the present invention will be detailed in the following paragraphs.

According to the present invention, a "luminescent metal complex" is a species comprising a lanthanide ion Ln capable of luminescence emission upon specific energy excitation, and an organic ligand L able to chelate the lanthanide ion in a thermodynamically stable and/or inert metal complex.

The lanthanide ion Ln is preferably in its oxidation state +3 or +2, i.e. in its cationic form Ln³⁺ or Ln²⁺.

When sensitized, i.e. when excited, the lanthanide ions are known to emit a characteristic luminescence, notably used in the fields of optical imaging.

Upon excitation, the lanthanide ion may emit in the visible, i.e. from 400 to 800 nm.

Upon excitation, the lanthanide ion may emit in the NIR, i.e. from 800 to 1600 nm.

Upon excitation, the lanthanide ion may emit both in the visible and the NIR.

According to one embodiment, the lanthanide metal Ln is selected from the group consisting of Ce, Pr, Nd, Sm, Eu, Gd, Tb, Dy, Ho, Er, Tm and Yb, preferably from the group consisting of Nd and Yb.

Nd and Yb, preferably in their cationic form Nd³⁺ and Yb³⁺, are luminescent lanthanides with emission bands in the NIR.

The spectrum of emission of Nd³⁺ appears as two main series of bands centered at 890 nm and 1065 nm.

The spectrum of emission of Yb³⁺ is centered around 980 nm.

According to the present invention, L is an organic ligand comprising a hydrophilic unit with at least 6 chelating groups chosen from the group consisting of hydroxyl, carboxyl, optionally in its carboxylate form, carbonyl, ether, phosphonate, phosphinate, thiolate, amino and imino functions.

According to the present invention, a "hydrophilic unit" is a part of a molecule with high affinity with water and with other hydrophilic molecules.

According to the present invention, a "chelating group" is a group capable of coordinating a metal species, preferably a metal cation. The organic ligand and the metal coordinated by said ligand together form a metal complex, which is stable under the conditions wherein the particles are maintained. The stability of the complex is needed for the relevance of the drug release monitoring.

The term "alkyl" means a saturated or unsaturated aliphatic hydrocarbon group which may be straight or branched having 1 to 24 carbon atoms in the chain. "Branched" means that one or lower alkyl groups such as methyl, ethyl or propyl are attached to a linear alkyl chain. The alkyl may be substituted with one or more "alkyl group substituents" which may be the same or different, and include for instance halo, cycloalkyl, aryl, hydroxyl, alkoxy, amino, carboxy.

The term "alkoxy" refers to an -O-alkyl radical, alkyl being defined above.

The term "cycloalkyl" as employed herein includes saturated cyclic, bicyclic, tricyclic, or polycyclic hydrocarbon groups having 3 to 12 carbons, wherein any ring atom capable of substitution may be substituted by a substituent. Examples of cycloalkyl moieties include, but are not limited to, cyclohexyl and adamantyl.

The term "halo" refers to the atoms of the group 17 of the periodic table (halogens) and includes in particular fluorine, chlorine, bromine, and iodine atom.

The term "aryl" (or Ar) refers to an aromatic monocyclic, bicyclic, or tricyclic hydrocarbon ring system, in C₆-C₁₀, wherein any ring atom capable of substitution may be substituted by a substituent. Examples of aryl moieties include, but are not limited to, phenyl, naphthyl, and anthracenyl.

The term "substituents" refers to a group "substituted" on an alkyl, heterocycle or aryl group at any atom of that group. Suitable substituents include, without limitation, alkyl, alkenyl, alkoxy, halo, hydroxy, cyano, nitro, amino, SO₃H, sulfate, sulfonate, phosphate, perfluoroalkyl, perfluoroalkoxy, carboxyl, oxo, imino (alkyl, aryl, aralkyl), amine, ester, amide, sulfonamide, aryl, heterocycle, and cycloalkyl.

The term "acyl" refers to an alkylcarbonyl, cycloalkylcarbonyl, arylcarbonyl, or heterocyclecarbonyl substituent, any of which may be further substituted by substituents.

The term "oxo" refers to an oxygen atom, which forms a carbonyl when attached to carbon, an N-oxide when attached to nitrogen, and a sulfoxide or sulfone when attached to sulfur.

The term "alkenyl" as employed herein includes partially unsaturated, nonaromatic, hydrocarbon groups having 2 to 12 carbons, preferably 2 to 6 carbons.

The present application discloses particles wherein the organic ligand L is a polyaminocarboxylate ligand, preferably comprising at least one, for example two, groups of formula -N[(CH₂)ᵢCOO⁻]₂, wherein i is from 1 to 6, or at least three groups of formula >N(CH₂)ⱼCOO⁻, wherein j is from 1 to 6.

Thanks to their amino and carboxylate chelating groups, the polyaminocarboxylate ligands are often used for coordinating metal cations.

The metal complexes corresponding to this embodiment are particularly stable and preserve the integrity of the lanthanide ion through time, thus avoiding toxicity that would be associated with *in vivo* lanthanide ion release.

The present application discloses particles wherein the organic ligand L comprises at least one group of formula -N= or -N<.

The non-binding doublet of the nitrogen atom of said group participates in the coordination of the lanthanide ion.

For example, the ligand may comprise an (aza)macrocycle, aromatic or nonaromatic, preferably comprising at least 4 chelating nitrogen-containing groups, such as -N= or -N<.

Examples of (aza)macrocycles are cyclens, cyclams, porphyrins, texaphyrins, phthalocyanins, and derivatives thereof.

Besides its hydrophilic unit, the organic ligand L preferably further comprises a lipophilic unit.

According to the present invention, a "lipophilic unit" is a part of a molecule with low affinity with water, and with high affinity with other lipophilic molecules such as alkanes, unsaturated hydrocarbon moieties, oils, fats, etc.

According to this embodiment, the organic ligand L is an amphiphilic molecule, i.e. a molecule comprising a hydrophilic unit and a lipophilic unit.

The lipophilic unit is preferably selected from linear or branched C₆-C₂₄ alkyl, C₆-C₂₄ alkoxy, and C₆-C₂₄ fluoroalkyl groups.

The lipophilic unit is preferably a C₁₂ alkoxy group.

According to the invention, the organic ligand L is of formula (I): 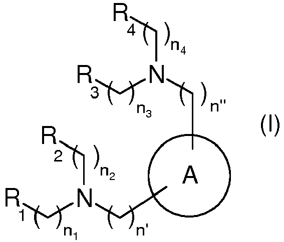 wherein:
- R₁, R₂, R₃ and R₄ are independently selected from the group consisting of hydroxyl, carboxyl, optionally in the carboxylate form, carbonyl, ether, phosphonate, phosphinate, thiolate, amino and imino functions, preferably from the group consisting of phosphonate and carboxylate functions,
- n₁, n₂, n₃ and n₄ are independently selected from 1 to 6,
- n' and n" are independently selected from 1 to 6,
- A is a saturated, partially saturated or aromatic heterocycle, comprising at least one coordinating group of formula -N= or -N<, said heterocycle being optionally substituted by one or more lipophilic groups, preferably selected from C₆-C₂₄ alkyl, C₆-C₂₄ alkoxy, and C₆-C₂₄ fluoroalkyl groups.

Preferably, n₁, n₂, n₃ and n₄ are 1.

Preferably, n' and n" are 1.

According to one embodiment, the organic ligand L is of formula (I'): 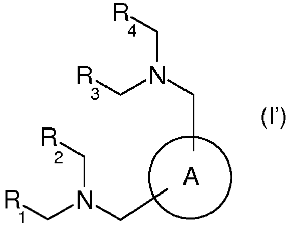 wherein R₁, R₂, R₃ and R₄ and A are defined as above.

The term "heterocycle" refers to a saturated 5-7 membered monocyclic, ring system having 1-3 heteroatoms, said heteroatoms being selected from O, N, or S (e. g. , carbon atoms and 1-3 heteroatoms of N, O, or S), wherein any ring atom capable of substitution may be substituted by a substituent.

Examples of "saturated heterocycle" include pyrrolidinyl, pyrazolidinyl, imidazolidinyl, oxiranyl, tetrahydrofuranyl, dioxolanyl, tetrahydro-pyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydropyranyl, tetrahydropyranyl, dihydropyranyl, tetrahydro-pyridyl, dihydropyridyl, tetrahydropyrinidinyl, dihydrothiopyranyl, azepanyl, as well as the fused systems resulting from the condensation with a phenyl group.

The term "heterocycle" also refers to an aromatic 5-8 membered monocyclic, 8-12 membered bicyclic, or 11-14 membered tricyclic ring system (also called heteroaryl) having 1-3 heteroatoms if monocyclic, 1-6 heteroatoms if bicyclic, or 1-9 heteroatoms if tricyclic, said heteroatoms selected from O, N, or S (e.g. , carbon atoms and 1-3, 1-6, or 1-9 heteroatoms of N, O, or S if monocyclic, bicyclic, or tricyclic, respectively), wherein any ring atom capable of substitution may be substituted by a substituent.

Examples of "aromatic heterocycle" include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, pyridyl-N-oxide , as well as the fused systems resulting from the condensation with a phenyl group.

According to one embodiment, the organic ligand L is of formula (II): 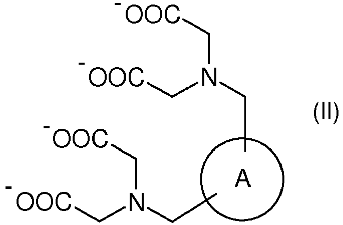 wherein A is as defined above.

In formulae (I), (I') and (II), A is preferably a C₄-C₁₀ saturated heterocycle comprising at least one nitrogen atom within the ring, such as a pyrrolidinyl, a piperidyl, a piperazinyl, a morpholinyl, or an azepanyl ring.

In formulae (I), (I') and (II), A is preferably a C₁-C₁₀ aromatic heterocycle, comprising at least one nitrogen atom within the ring, such as a pyrrolyl, an imidazolyl, a pyrazolyl, a pyridyl, or an indolyl ring.

According to one embodiment, A is a nitrogen-containing aromatic heterocycle ring in C₁-C₅, optionally substituted.

A is preferably a pyridyl ring, optionally substituted.

According to one embodiment, the organic ligand L is of formula (III): 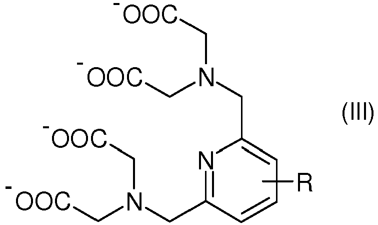 wherein R is selected from the group consisting of linear or branched C₆-C₂₄ alkyl, C₆-C₂₄ alkoxy, and C₆-C₂₄ fluoroalkyl groups.

R is preferably a lipophilic unit.

R is for example a C₆-C₂₄ alkoxy group, preferably a -OC₁₂H₂₅ group.

The organic ligand L is for example the ligand pyOC12 which has been synthesized as previously described (Bonnet et al. Chem. Commun. 2010, 46, 124):

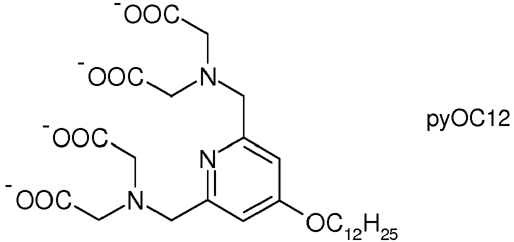

According to the invention, the organic ligand L is of formula (1): 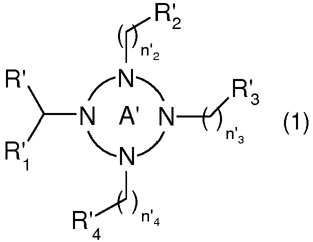 wherein:
- R'₁, R'₂, R'₃ and R'₄ are independently selected from the group consisting of hydroxyl, carboxyl, optionally in the carboxylate form, carbonyl, ether, phosphonate, phosphinate, thiolate, amino and imino functions,
- n'₁, n'₂ and n'₃ are independently selected from 1 to 6,
- R' is selected from the group consisting of hydrogen, methyl, linear or branched C₂-C₂₄ alkyl groups, and C₁-C₂₄ fluoroalkyl groups, and
- A' is a saturated tetraazacycloalkyl ring comprising from 6 to 12 carbon atoms.

In formula (1), A' is preferably a cyclen ring (1,4,7,10-tetraazacyclododecane) or a cyclam ring (1,4,8,11-tetraazacyclotetradecane).

In formula (1), n'₁, n'₂ and n'₃ are preferably 1.

According to one embodiment, the organic ligand L is of formula (1'): 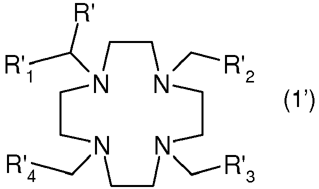 wherein R'₁, R'₂, R'₃ and R'₄ and R' are defined as above.

In formulae (1) and (1'), R'₁, R'₂, R'₃ and R'₄ are preferably carboxylate functions. According to one embodiment, the organic ligand L is of formula (2): 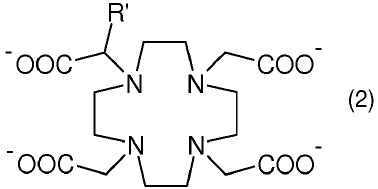 wherein R' is as defined above.

In formulae (1), (1') and (2), R' is preferably a lipophilic unit.

In formulae (1), (1') and (2), R' is preferably a linear or branched C₆-C₂₄ alkyl group, for example a -C₁₂H₂₅ group.

The organic ligand L is for example the ligand DOTAC12 which has been synthesized as previously described (Nicolle et al. J. Biol. Inorg. Chem. 2002, 7, 757):

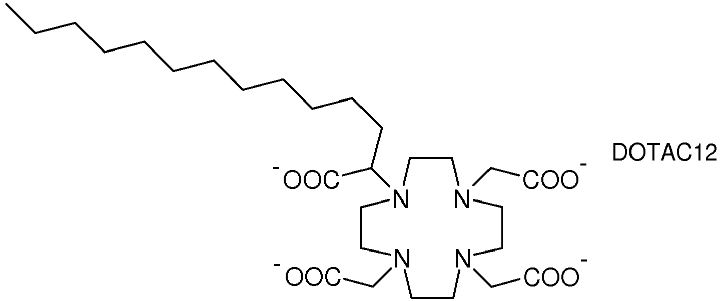

According to one embodiment, the Ln/drug molar ratio in the particles is from 1:1 to 10:1, preferably from 1:1 to 5:1, more preferably from 2:1 to 4:1, and is typically about 3:1.

Said ranges of ratio enable a luminescence suitable for the monitoring via optical means.

Many kinds of drugs may be used in the framework of the present invention, as long as they incorporate a conjugated aromatic system.

According to the present invention, a "drug" is a therapeutic agent, useful for the treatment and/or the prevention of a disease and/or a condition.

The drug may be amphiphilic, lipophilic or hydrophilic.

When the drug is lipophilic, it is typically located within a lipophilic zone of the particle, such as in the lipidic bilayer of a liposome or in the lipidic core of a lipoparticle.

When the drug is hydrophilic, it is typically located within a hydrophilic zone of the particle, such as the aqueous core of a liposome.

When the drug is amphiphilic, it may be located either within a hydrophilic zone of the particle, or within a lipophilic zone of the particle, or both within a hydrophilic zone and a lipophilic zone of the particle.

As mentioned above, the drug inside the particles of the invention comprises a conjugated aromatic system. The inventors have observed that said system is able of absorbing excitation energy and transferring it to the metal complex. It seems that the drug according to the invention is a chromophore, acting as antenna, able to sensitize the luminescence of the lanthanide.

According to one embodiment, the conjugated aromatic system is selected from heterocyclic and non-heterocyclic moieties, including anthracene, naphthalene, naphthalimide, anthraquinone, naphthoquinone, quinoline, isoquinoline, indole, quinazoline, purine, and derivatives thereof, preferably from anthraquinone, quinoline and derivatives thereof.

A heterocyclic conjugated aromatic system is a system wherein at least one heteroatom, such as a nitrogen, an oxygen or a sulfur atom, participates in the conjugation system. Quinoline and anthraquinone are examples of heterocyclic conjugated aromatic system.

A non-heterocyclic conjugated aromatic system is a system wherein only carbon atoms are engaged in the conjugation system. Anthracene is an example of non-heterocyclic conjugated aromatic system.

By "derivatives", it is meant that the conjugated system may be further substituted by one or more substituents, such as C₁-C₆ alkyl groups, C₁-C₆ alkoxy groups, or C₁-C₆ aminoalkyl groups, said substituents being optionally substituted.

Preferably, the conjugated aromatic system of the drug is selected from anthraquinone, quinoline and derivatives thereof.

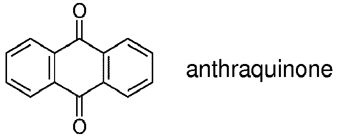

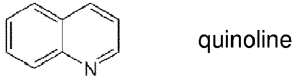

According to one embodiment, the drug is an anticancer agent chosen from the group consisting of doxorubicin, mitoxantrone and irinotecan.

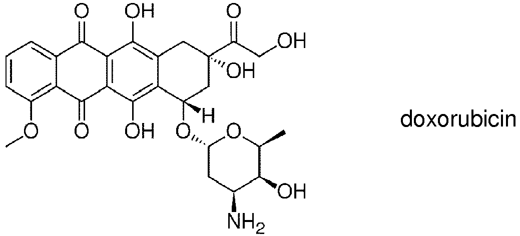

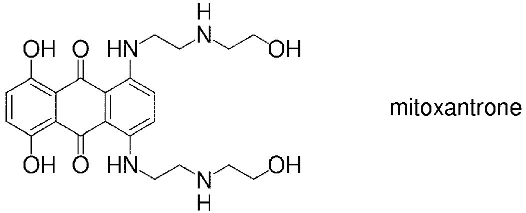

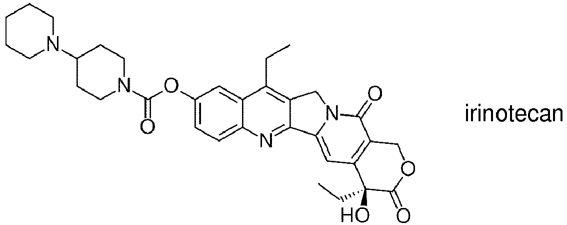

The wavelength of excitation of the drug according to the invention is preferably from 250 nm to 800 nm, more preferably from 400 nm to 800 nm, advantageously from 600 nm to 800 nm.

For example, the wavelengths of excitation of doxorubicin are from 300 nm to 650 nm, the wavelengths of excitation of mitoxantrone from 500 nm to 750 nm, and the wavelengths of excitation of irinotecan are from 300 nm to 500 nm.

According to another embodiment, the particle of the invention further comprises a chromophore, different from the drug.

An alternate strategy to trigger a change of luminescence signal upon drug delivery is based on its use as a "quencher". According to this embodiment, the drug no longer acts as an antenna but as a "quencher", which results in the inhibition of the lanthanide luminescence. "Quenching" refers to any process which results in the decrease of the luminescence intensity of the lanthanide ion.

In this mode, the lanthanide ion is sensitized with a different antenna than the drug which is present in the particle of the invention.

In this setup, the drug acts as a "quencher" and induces the absence of signal or the presence of a weaker signal when the drug is in the particles. Upon excitation, the energy absorbed by said chromophore is deactivated by the drug, so that the lanthanide of the metal complex is not sensitized or not sensitized efficiently. Thus, when the particle is intact, the lanthanide ion does not emit any luminescence, or only a low luminescence.

Upon release of the drug from the particle, the "quenching effect" induced by the drug disappears and the energy absorbed by the chromophore can be transferred to the lanthanide. The luminescence of the lanthanide ion is thus detectable or is more intense. The particle is thus more detectable once the drug is released from said particle.

Many kinds of particles may be used in the framework of the present invention, such as core/shell particles, liposomes, lipoparticles, coated solid particles, particles obtained via polymerization, gelified particles, hollow particles, or particles resulting from sonication.

According to one embodiment, the particles are core/shell particles.

According to the present invention, a "core/shell particle" is a particle formed of a core surrounded by a shell.

The shell may be a liposomal surfactant layer, or a polymerized shell, or a gelified envelope. The core may be solid, liquid, or gelified.

According to one embodiment, the particles are lipoparticles.

According to the present invention, a "lipoparticle" is a particle formed of an aqueous core surrounded by a phospholipid monolayer.

According to one embodiment, the particles are liposomes.

According to the present invention, a "liposome" is a particle formed of an aqueous core surrounded by a phospholipid bilayer.

The phospholipid bilayer is a thin polar membrane made of two layers of phospholipid molecules. These membranes are flat sheets that form a continuous barrier around liposomes. The phospholipid bilayer is the barrier that prevents the core of the liposomes from diffusing into areas where they should not be. Phospholipid bilayers are ideally suited to this role because, even though they are only a few nanometers in width, they are impermeable to most hydrophilic molecules.

Phospholipids have a hydrophilic head and two hydrophobic tails each. When phospholipids are exposed to water, they arrange themselves into a two-layered sheet (a bilayer) with all of their tails pointing toward the center of the sheet. The center of this bilayer contains almost no water and excludes hydrophilic molecules.

The phospholipid bilayer typically includes several types of lipids other than phospholipids, such as targeting agents able to specifically target cellular receptors.

The aqueous core of the liposome is preferably liquid.

The release of the drug from a liposome may occur upon rupture of said liposome. Alternatively, the drug may also be released from a liposome without breaking said liposome.

In the case of a liposome, the metal complex is preferably amphiphilic.

Accordingly, the metal complex is preferably located within the phospholipid bilayer of the liposome.

In the case of a liposome comprising phospholipids and wherein the metal complex is located within the phospholipid bilayer, the molar ratio Ln:phospholipids is preferably about 1:1.

The phospholipids forming the bilayer of the liposomes are preferably selected from the group consisting of phosphatidylcholine, phosphatidylserine, phosphatidylinositol, phosphatidylglycerol, diphosphatidylglycerol and N-acyl phosphatidylethanolamine, dioleoyl phosphatidylcholine, dipalmitoyl phosphatidylcholine, distearoyl phosphatidylcholine, dimyristoyl phosphatidylcholine, 1-palmitoyl-2-oleoyl-sn-glycero-3-phosphocholine, 1-oleoyl-2-palmitoyl-sn-glycero-3-phosphocholine, 1,2-dioleoyl-sn-glycero-3-[phospho-rac-(1-glycerol)], 1,2-dipalmitoyl-sn-glycero-3-[phospho-rac-(1-glycerol)], 1,2-distearoyl-sn-glycero-3-[phospho-rac-(1-glycerol)], 1,2-dimyristoyl-sn-glycero-3-[phospho-rac-(1-glycerol)], 1-palmitoyl-2-oleoyl-sn-glycero-3-[phospho-rac-(1-glycerol)], 1-oleoyl-2-palmitoyl-sn-glycero-3-[phospho-rac-(1-glycerol)], N-decanoyl phosphatidylethanolamine, N-undecanoyl phosphatidylethanolamine, N-dodecanoyl phosphatidylethanolamine, N-tridecanoyl phosphatidylethanolamine, and N-tetradecanoyl phosphatidylethanolamine, and mixtures thereof.

Preferably, the phospholipid is dimyristoyl phosphatidylcholine (DMPC).

According to one particular embodiment, the particles of the present invention are liposomes, comprising an aqueous core surrounded by a phospholipid bilayer, said bilayer comprising at least one amphiphilic luminescent metal complex as defined above.

The lanthanide ion according to this embodiment is preferably Nd³⁺ and/or Yb³⁺.

Depending on their lipophilic of hydrophilic properties, the incorporated drug molecules may be localized either in the bilayer or in the aqueous core of the liposomes, said aqueous core being preferably liquid.

According to a first variant of this embodiment, the drug is hydrophilic and therefore located in the aqueous core of the liposomes, preferably homogeneously distributed within the core. According to this variant, the drug molecules able to sensitize the metal complex are located in the zone of the core that is in contact with the bilayer.

According to a second variant of this embodiment, the drug is lipophilic and therefore located in the bilayer of the liposomes, preferably homogeneously distributed within the bilayer. According to this variant, both the drug molecules and the metal complexes are distributed in the bilayer and are thus in close proximity, thus allowing the sensitization of the metal complex.

The ligand L according to this particular embodiment is preferably of formula (I) or (II), wherein A is substituted by a lipophilic unit, or of formula (III), wherein R is a lipophilic unit, or of formula (1), (1') or (2), wherein R' is a lipophilic unit.

For example, L is pyOC12 or DOTAC12 as defined above.

For example, the luminescent metal complex is selected from the group consisting of Nd-pyOC12, Yb-pyOC12, and a mixture thereof.

For example, the luminescent metal complex is Nd-DOTAC12.

The particles of the invention preferably further comprise a targeting agent on the surface of said particles, preferably selected from the group consisting of a protein (preferably an apolipoprotein), a synthetically modified protein, a genetically modified protein, a natural or synthetic peptide, a saccharide, a natural or synthetic oligonucleotide, an aptamer, a small synthetic vector (preferably folic acid), capable of targeting cellular receptors.

A liposome further comprising on its surface a protein able to specifically targeting receptors is also called a "lipoprotein". In respect to liposomes, lipoprotein particles offer further advantages for biological targeting.

The targeting agent may be grafted onto the particle surface after its formation, typically via covalent chemical grafting using methods known in the art (see P. Caravan, Z. Zhang, Targeted MRI contrast agents in The Chemistry of Contrast Agents in Medical MRI, 2nd Edition, Eds. A. E. Merbach, L. Helm, E. Toth, Wiley, 2013, Chichester).

In the case of liposomes, the targeting agent may be grafted onto a phospholipid prior to the liposome formation. The hereby obtained "grafted phospholipid" is intended to be incorporated within the bilayer of the liposome, exhibiting its targeting agent on the surface of the liposome.

The particles of the invention have a mean size comprised from 3 nm to 2 000 nm, preferably from 5 nm to 1 000 nm, preferably from 10 nm to 500 nm, more preferably from 10 nm to 300 nm, advantageously from 10 nm to 150 nm, for example from 10 nm to 100 nm.

The mean size of the particles of the invention is typically measured by Dynamic Light Scattering (DLS), in a Mavern Zetasizer Nano Series equipment, at 20.0°C.

The present invention also relates to the particles as defined above, wherein the drug is an anticancer agent, for use for treating cancer.

The present invention also relates to the particles as defined above for use in imaging methodologies.

The present invention also relates to a method of treating cancer, comprising the step of administering to a patient in need thereof the particles as defined above in a pharmaceutically acceptable excipient, wherein the drug is an anticancer agent.

Optionally, said method further comprises the monitoring of the release of the drug comprised in the particles, preferably by NIR detection.

The present invention also relates to a method of treating cancer, comprising the step of administering to a patient in need thereof a pharmaceutical composition comprising at least one particle as defined above and at least one pharmaceutically acceptable excipient, wherein the drug is an anticancer agent.

The present invention particularly relates to the particles as defined above for the use in imaging methodologies, for monitoring the release of the drug, preferably by NIR optical detection.

According to the particles of the present invention, the loss of luminescence means that the drug, originally incorporated in the particles, has been released from said particles, or else that the particles have been broken, both corresponding to the release of the drug to its site of interest.

The NIR optical detection of the metal complex can be visualized by optical means, using a macroscope Nikon AZ100 Multizoom equipped with a camera Evolve 512 EMCCD.

In the case of particles further incorporating Gd³⁺ complex, the monitoring of the particles may be achieved via MRI detection of the Gd³⁺ complex, using classical T1 mapping imaging techniques (see B-T Doan, S. Même, J-C. Beloeil, General principles of MRI in The Chemistry of Contrast Agents in Medical MRI, 2nd Edition, Eds. A. E. Merbach, L. Helm, E. Toth, Wiley, 2013, Chichester).

The present invention also relates to a pharmaceutical composition comprising at least one particle as defined above and at least one pharmaceutically acceptable excipient.

"Pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

The pharmaceutical compositions can be administered in a suitable formulation to humans and animals by topical or systemic administration, including oral, rectal, nasal, buccal, ocular, sublingual, transdermal, rectal, topical, vaginal, parenteral (including subcutaneous, intra-arterial, intramuscular, intravenous, intradermal, intrathecal and epidural), intracisternal and intraperitoneal. It will be appreciated that the preferred route may vary with for example the condition of the recipient.

The present invention also relates to a method for preparing the liposomes as defined above, comprising the steps of:
- preparing a solution comprising an organic solvent, the luminescent metal complex as defined above and a phospholipid,
- evaporating the organic solvent in order to obtain a lipidic film,
- forming liposomes, typically by hydrating said film and sonicating the resulting mixture, and
- loading the drug as defined above into said liposomes, preferably by incubating the liposomes in a solution of said drug.

The method of preparation of the liposomes is adapted from the method described in Cormode et al. Small, 2008, 4: 1437.

The molar ratio Ln:phospholipids in the solution is preferably about 1:1.

The organic solvent may be a mixture of organic solvents, comprising preferably polar solvents, such as methanol, chloroform, acetonitrile, DMSO (dimethyl sulfoxide), DMF (dimethylformamide).

Advantageously, the solution is sonicated, for example for 30 min, for example at 37°C.

The organic solvent or mixture of solvents is then evaporated, preferably under a N2 flow in order to obtain a lipidic film.

Said film is then typically hydrated with an aqueous solution, such as a buffer of acidic pH (for example NH₄OAc buffer, pH 4.8), in order to obtain a suspension, which is preferably sonicated at low temperature, typically at 10°C.

The liposomes thus obtained may be further incubated in a solution of neutral pH (such as PBS medium, pH 7.4) in order to create a pH gradient useful for loading the desired drug molecule.

The loading of said drug is then carried out by transferring the unloaded liposomes into a solution of said drug. If the drug is hydrophilic, said solution is an aqueous solution. If the drug is lipophilic, said solution is a lipidic solution. If the drug is amphiphilic, said solution is preferably an aqueous solution.

For example, in the case of doxorubicin, mitoxanthrone or irinotecan, the liposomes are added in an aqueous solution of the respective drug and then stirred for several hours at room temperature to enable the loading.

After the loading of the drug, the liposomes may be dialysed, i.e incubated in an aqueous solution to remove any free drug.

In the case of the synthesis of lipoproteins, the targeting agent is further incorporated by incubating the liposomes with the targeting agent (such as an apolipoprotein), followed by a sonication and a final dialysis step.

### Experimental Part

### Materials

The ligand pyOC12 has been synthesized as previously described (Bonnet et al. Chem. Commun. 2010, 46, 124). The ligand DOTAC12 has been synthesized as previously described (Nicolle et al. J. Biol. Inorg. Chem. 2002, 7, 757).GdCl₃.6H₂O, YbCl₃, NdCl₃.6H₂O, DMPC (1,2-dimyristoyl-*sn*-glycero-3-phosphocholine), Doxorubicin, Mitoxantrone, Irinotecan, n-octyl-glucoside, PBS (0.05% Tween 20, pH 7.4), methanol and chloroform were purchased from Sigma-Aldrich. ICP Gd³⁺ standard (10000 mg/L in 5% HNO₃) was purchased from Spex CertiPrep. ApoE4 was purchased from AbCam. All the products have been used without further purification, unless stated otherwise.

### Synthesis of the particles

Liposomes and lipoproteins containing different luminescent lanthanide ions were synthesised by the lipid film method, adapted from the literature (Cormode et al. Small, 2008, 4: 1437). In some preparations, Gd³⁺ complexes have been also integrated in the preparation to make the particles also detectable in MRI.

Briefly, an equimolar amount of phospholipid (DMPC) and metal complex (Nd-pyOC12, Yb-pyOC12 or Nd-DOTAC12) are solubilised in MeOH/CHCl₃ and sonicated for 30 min at 37°C. After this time, the solvents are evaporated under a N2 flow and the lipidic film formed is then hydrated with NH₄OAc buffer, pH 4.8. This suspension is incubated at 37°C for 1h and follow by a sonicated for 30min at 10°C. The final solution is dialysed against PBS (3.5KDa cut-off membrane), allowing the formation of "acidic" particles, in PBS pH 7.4 medium (and therefore creating a pH gradient). The desired drug molecule (doxorubicin, mitoxanthrone or irinotecan) is then added in a 3:1 total Ln/drug molar ratio and stirred for 7h at room temperature.

After the incorporation of the drug, the particles are once again dialysed against PBS to remove any free drug.

In the case of the synthesis of lipoproteins, after the doxorubicin encapsulation step, the ApoE4 (1 mg ApoE4/ 5 mg lipids) is incubated for 1h at 37°C followed by a sonication of 20min at 10°C and the final dialysis step against PBS.

### Measurements

Induced Coupled Plasma Optical Emission Spectrometric (ICP-OES) measurements have been performed to determine the lanthanide concentration in each particle. The particles formed were analyzed by Dynamic Light Scattering (DLS) to acquire about their size (∼12-50 nm in diameter), in a Mavern Zetasizer Nano Series equipment, at 20.0°C.

The encapsulation efficiency of the drug has been determined by UV-vis absorption, upon lysis of the particles with n-octyl-glucoside (40%).

### ICP-OES measurements

Induced Coupled Plasma Optical Emission Spectrometer (ICP-OES) measurements were performed in a Jobin Yvon ULTIMA2 Spectrometer. Both standards solutions of the corresponding Ln³⁺ and the samples were prepared in 5% HNO₃. The measurements were performed using the 3 characteristic bands of Gd^{III}, being the most accurate results those corresponding to the 342.246 nm band for Gd^{III}. All the measurements were done in triplicate.

### Photophysical measurements

Absorbance UV spectra were performed on a Secomam Uvikon XL spectrophotometer. Emission and excitation spectra were measured using a Jobin-Yvon Horiba Fluorolog-322 spectrofluorimeter equipped with a Hamamatsu R928 detector for UV-vis detection and an InGaAs Jobin-Yvon FL-1046 detector for the NIR region detection. Emission and excitation spectra were recorded using a 700 nm cut-off filter. Spectra were corrected for instrumental response due to the excitation lamp output, spectral responses of the excitation and emission gratings, response of the detector, and the use of neutral density filters, when applicable.

### Results

The following liposomes were prepared:
(A) DMPC/Nd-pyOC12/Yb-pyOC12/doxorubicin
(B) DMPC/Nd-pyOC12/doxorubicin
(C) DMPC/Yb-pyOC12/doxorubicin
(D) DMPC/Nd-pyOC12/mitoxantrone
(E) DMPC/Yb-pyOC12/irinotecan
(F) DMPC/Nd-DOTAC12/doxorubicin

The following lipoprotein was prepared:
(A') DMPC/Nd-pyOC12/Yb-pyOC12/doxorubicin+ApoE4

Liposomes (A) to (E) and lipoprotein (A') were also prepared by incorporating Gd-pyOC12.

The results of absorption/emission measurements are as follows:

| | (A) | (B) | (C) | (D) | (E) | (F) |
|---|---|---|---|---|---|---|
| λ_{excitation} (nm) | 495 - 600 | 495 - 600 | 495 - 600 | 500 - 700 | 370 | 495-600 |
| λₑₘᵢₛₛᵢₒₙ (nm) | 890 ^{(lb)} | 890 ^{(lb)} | 980 ^{(lb)} | 890 ^{(lb)} | 980 ^{(lb)} | 890 ^{(lb)} |
| | 980 ^{(lb)} | | | | | |
| | 1064 ^{(sb)} | 1064 ^{(sb)} | | 1064 ^{(sb)} | | 1064 ^{(sb)} |

| | | | | | | |
|---|---|---|---|---|---|---|
| ^{(lb)} large band ^{(sb)} sharp band | | | | | | |

Upon excitation (notably at 495, 543, 585 and 600 nm), liposomes (A) exhibited luminescence centred mainly at 890 nm and 1064 nm, which is characteristic of Nd³⁺ luminescence, and also exhibited luminescence centred mainly at 980 nm, which is characteristic of Yb³⁺ luminescence.

Upon excitation (notably at 495, 543, 585 and 600 nm), liposomes (B) exhibited luminescence centred mainly at 890 and 1064 nm, which is characteristic of Nd³⁺ luminescence.

Upon excitation (notably at 495, 543, 585 and 600 nm), liposomes (C) exhibited luminescence centred mainly at 980 nm, which is characteristic of Yb³⁺ luminescence.

Upon excitation (notably at 600 nm and 660 nm), liposomes (D) exhibited luminescence centred mainly at 890 nm and 1064 nm, which is characteristic of Nd³⁺ luminescence.

Upon excitation (notably at 370 nm), liposomes (E) exhibited luminescence centred mainly at 980 nm, which is characteristic of Yb³⁺ luminescence.

Upon excitation (notably at 495, 543, 585 and 600 nm), liposomes (F) exhibited luminescence centred mainly at 890 nm and 1064 nm, which is characteristic of Nd³⁺ luminescence.

The luminescence studies of the doxorubicin, mitoxantrone and irinotecan containing liposomes and lipoproteins showed that these drug molecules are capable of sensitizing both Nd³⁺ and Yb³⁺ luminescence in the near IR (excitation at 370 nm for irinotecan, 495 to 600 nm for doxorubicin and 500-700 nm for mitoxantrone).

It was observed that for these drugs used as chromophores, the absorption and the excitation spectra of the particles recorded upon lanthanide emission have fairly similar profiles. This result shows that the electronic structure centred on the chromophore drugs is indeed used for the sensitization of both Nd³⁺ and Yb³⁺.

A further proof that the sensitization of the metal complexes incorporated in the particles is indeed achieved by the "antenna effect" of the drug molecules, n-octyl-glucoside was added to the particles, a detergent known to induce liposome disruption.

Upon addition of 40% of n-octyl-glucoside to a batch of liposomes (C) or (F), a complete loss of the Yb³⁺ luminescence (band centred at 985 nm) and an increase of the doxorubicin emission (bands centred at 550 and 590 nm) are observed. After liposome destruction, the energy received by the doxorubicin is no longer transferred to the lanthanide.

This result also shows that the particles according to the invention, involving luminescent metal complexes, allow for a direct follow-up of drug release.

### Results in vivo

A NIR imaging device optimized for the detection of NIR emitting Ln³⁺ reporters from cells to small animals has been created.

First, liposomes (C) were internalized in HeLa cells. The luminescence arising from the Yb³⁺ NIR emission was detected with a good signal-to-noise ratio.

Further, tumors were induced in female BALB/c mice by subcutaneous injection of 10⁶ 4T1 murine mammal carcinoma cells. After tumor growth (15 days), liposomes (C) were injected into the tumor (∼5 µM doxorubicin concentration). For comparison, the same concentration of doxorubicin was injected in control mice.

Macroscopy images of the tumor region on mice were obtained using a macroscope Nikon AZ100 Multizoom equipped with a camera Evolve 512 EMCCD.

For detection of the Yb³⁺, the following set of filters was used: λ_{excitation}: 482/35nm, dichroic 705, λₑₘᵢₛₛᵢₒₙ: LP770nm.

For the detection of doxorubicin signal, the following set of filters was used: λ_{excitation}: 447/60nm, dichroic 605, λₑₘᵢₛₛᵢₒₙ: 536/40nm.

In the visible region, a very intense autofluorescence is observed preventing the detection of the signal originating from the imaging probe.

In the NIR region, the doxorubicin has a weak emission signal, while the Yb³⁺ NIR emission arising from the liposomes can be detected with a short acquisition time.

Indeed, in the NIR, the background luminescence is extremely weak or non-existent, favouring a good signal-to-noise ratio and detection sensitivity.

Those results demonstrate the feasibility of lanthanide-based NIR imaging in living cells and also in living mice with liposomes (C).

The particles of the invention thus enable the acquisition of optical images by detecting non-invasively the drug-sensitized lanthanide luminescence in living mice.

This work establishes a proof of principle that, despite their modest quantum yields, NIR emitting lanthanide ions in the particles of the invention can emit a sufficient number of photons to allow sensitive bio-imaging using drug-sensitized lanthanide luminescence in the NIR.

## Claims

1. Particle comprising :
- at least one luminescent metal complex of formula [Ln].[L], wherein:
- Ln is a lanthanide ion,
- L is an organic ligand comprising a hydrophilic unit with at least 6 chelating groups chosen from the group consisting of hydroxyl, carboxyl, optionally in the carboxylate form, carbonyl, ether, phosphonate, phosphinate, thiolate, amino and imino functions, and
- at least one drug comprising a conjugated aromatic system, wherein said drug is not covalently bonded to the metal complex, and wherein the drug is an anticancer agent,
said particle having a size comprised from 3 nm to 2 000 nm, and
wherein the distribution of the metal complex and the drug within the particle is such that the mean distance between a drug molecule and a metal complex is less than 15 nm,
wherein said ligand is of formula (I): 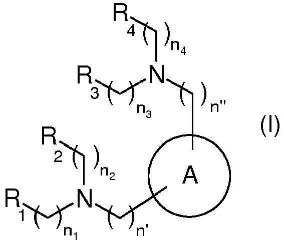 wherein:
- R₁, R₂, R₃ and R₄ are independently selected from the group consisting of hydroxyl, carboxyl, optionally in the carboxylate form, carbonyl, ether, phosphonate, phosphinate, thiolate, amino and imino functions,
- n₁, n₂, n₃ and n₄ are independently selected from 1 to 6,
- n' and n" are independently selected from 1 to 6, and
- A is a saturated, partially saturated or aromatic heterocycle, comprising at least one coordinating group of formula -N= or -N<, said heterocycle being optionally substituted by one or more lipophilic groups,
or wherein said ligand is of formula (1): 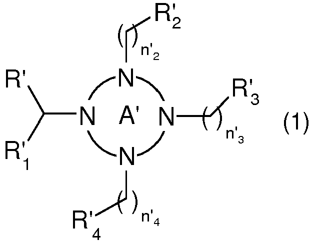 wherein:
- R'₁, R'₂, R'₃ and R'₄ are independently selected from the group consisting of hydroxyl, carboxyl, optionally in the carboxylate form, carbonyl, ether, phosphonate, phosphinate, thiolate, amino and imino functions,
- n'₁, n'₂ and n'₃ are independently selected from 1 to 6,
- R' is selected from the group consisting of hydrogen, methyl, linear or branched C₂-C₂₄ alkyl groups, and C₁-C₂₄ fluoroalkyl groups, and
- A' is a saturated tetraazacycloalkyl ring comprising from 6 to 12 carbon atoms.

2. Particle according to claim 1, wherein the lanthanide is selected from the group consisting of Ce, Pr, Nd, Sm, Eu, Gd, Tb, Dy, Ho, Er, Tm and Yb.

3. Particle according to anyone of claim 1 or 2, wherein the drug is chosen from the group consisting of doxorubicin, mitoxantrone and irinotecan.

4. Particle according to anyone of claims 1 to 3, **characterized in that** it is chosen from the group consisting of a core/shell particle, a liposome, a lipoparticle, a coated solid particle, a particle obtained via polymerization, a gelified particle, a hollow particle, and a particle resulting from sonication.

5. Particle according to claim 4, **characterized in that** it is a liposome comprising an aqueous core surrounded by a phospholipid bilayer.

6. Particle according to any one of claims 1 to 5, for use in the treatment of cancer.

7. Particle according to anyone of claims 1 to 5, for use in imaging methodologies.

8. Pharmaceutical composition comprising at least one particle according to anyone of claims 1 to 5, and at least one pharmaceutically acceptable excipient.

9. Method for preparing the particle according to claim 5, comprising the steps of:
- preparing a solution comprising an organic solvent, the luminescent metal complex as defined in claim 1 and a phospholipid,
- evaporating the organic solvent in order to obtain a lipidic film,
- forming liposomes, typically by hydrating said film and sonicating the resulting mixture, and
- loading the drug as defined in claim 1 into said liposomes, preferably by incubating the liposomes in a solution of said drug.

## Patentansprüche

1. Partikel, umfassend:
- mindestens einen lumineszierenden Metallkomplex der Formel [Ln].[L], wobei:
- Ln eine Lanthanoid-Ion ist,
- L ein organischer Ligand ist umfassend eine hydrophile Einheit mit mindestens 6 chelatisierenden Gruppen ausgewählt aus der Gruppe bestehend aus Hydroxyl-, Carboxyl-, wahlweise in der Carboxylat-Form, Carbonyl-, Ether-, Phosphonat-, Phosphinat-, Thiolat-, Amino- und Imino-Funktionen, und
- mindestens einen Wirkstoff umfassend ein konjugiertes aromatisches System, wobei der Wirkstoff nicht kovalent an den Metallkomplex gebunden ist und wobei der Wirkstoff ein Antikrebswirkstoff ist,
wobei das Partikel eine Größe von 3 nm bis 2000 nm hat und
wobei die Verteilung des Metallkomplexes und des Wirkstoffs in dem Partikel derart ist, dass die mittlere Entfernung zwischen einem Wirkstoffmolekül und einem Metallkomplex weniger als 15 nm beträgt,
wobei der Ligand die Formel (I) hat: 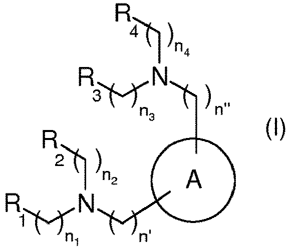 wobei:
- R₁, R₂, R₃ und R₄ unabhängig ausgewählt sind aus der Gruppe bestehend aus Hydroxyl-, Carboxyl-, wahlweise in der Carboxylat-Form, Carbonyl-, Ether-, Phosphonat-, Phosphinat-, Thiolat-, Amino- und Imino-Funktionen,
- n₁, n₂, n₃ und n₄ unabhängig ausgewählt sind aus 1 bis 6,
- n' und n" unabhängig ausgewählt sind aus 1 bis 6, und
- A ein gesättigter, teilweise gesättigter oder aromatischer Heterozyklus ist, umfassend mindestens eine Koordinationsgruppe der Formel -N= oder -N<, wobei der Heterozyklus wahlweise substituiert ist mit einer oder mehreren lipophilen Gruppen,
oder wobei der Ligand die Formel (1) hat: 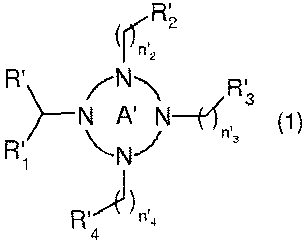 wobei:
- R'₁, R'₂, R'₃ und R'₄ unabhängig ausgewählt sind aus der Gruppe bestehend aus Hydroxyl-, Carboxyl-, wahlweise in der Carboxylat-Form, Carbonyl-, Ether-, Phosphonat-, Phosphinat-, Thiolat-, Amino- und Imino-Funktionen,
- n'₁, n'₂ und n'₃ unabhängig ausgewählt sind aus 1 bis 6,
- R' ausgewählt ist aus der Gruppe bestehend aus Wasserstoff, Methyl, linearen oder verzweigten C₂-C₂₄ Alkylgruppen und C₁-C₂₄ Fluoralkylgruppen, und
- A' ein gesättigter Tetraazacycloalkylring umfassend 6 bis 12 Kohlenstoffatome ist.

2. Partikel gemäß Anspruch 1, wobei das Lanthanoid ausgewählt ist aus der Gruppe bestehend aus Ce, Pr, Nd, Sm, Eu, Gd, Tb, Dy, Ho, Er, Tm und Yb.

3. Partikel gemäß einem von Anspruch 1 oder 2, wobei der Wirkstoff ausgewählt ist aus der Gruppe bestehend aus Doxorubicin, Mitoxantron und Irinotecan.

4. Partikel gemäß einem der Ansprüche 1 bis 3, **gekennzeichnet dadurch, dass** es ausgewählt ist aus der Gruppe bestehend aus einem Kern/Schale-Partikel, einem Liposom, einem Lipopartikel, einem beschichteten festen Partikel, einem Partikel erhalten durch Polymerisation, einem gelierten Partikel, einem hohlen Partikel und einem Partikel resultierend aus der Ultraschallbehandlung.

5. Partikel gemäß Anspruch 4, **gekennzeichnet dadurch, dass** es ein Liposom umfassend einen wässrigen Kern umgeben von einer Phospholipid-Doppelschicht ist.

6. Partikel gemäß einem der Ansprüche 1 bis 5, zur Verwendung in der Behandlung von Krebs.

7. Partikel gemäß einem der Ansprüche 1 bis 5, zu Verwendung in bildgebenden Verfahren.

8. Pharmazeutische Zusammensetzung umfassend mindestens ein Partikel gemäß einem der Ansprüche 1 bis 5 und mindestens einen pharmazeutisch annehmbaren Trägerstoff.

9. Verfahren zur Herstellung des Partikels gemäß Anspruch 5, umfassend dir Schritte des:
- Herstellens einer Lösung umfassend ein organisches Lösemittel, den lumineszierenden Metallkomplex wie in Anspruch 1 definiert und ein Phospholipid,
- Verdampfens des organischen Lösemittels, um einen Lipid-Film zu erhalten,
- Bildens von Liposomen, typischerweise durch Hydratisieren des Films und Beschallen der resultierenden Mischung, und
- Einschleusens des Wirkstoffs wie in Anspruch 1 definiert in die Liposomen, vorzugsweise durch Inkubieren der Liposomen in einer Lösung des Wirkstoffs.

## Revendications

1. Particule comprenant :
- au moins un complexe métallique luminescent de formule [Ln]•[L] dans laquelle :
- Ln est un ion lanthanide,
- L est un ligand organique comprenant un motif hydrophile avec au moins 6 groupes chélatants choisis dans l'ensemble constitué par les fonctionnalités hydroxyle, carboxyle, éventuellement sous forme carboxylate, carbonyle, éther, phosphonate, phosphinate, thiolate, amino et imino, et
- au moins un médicament comprenant un système aromatique conjugué, lequel médicament n'est pas lié de manière covalente au complexe métallique, et lequel médicament est un agent anticancéreux,
ladite particule ayant une taille comprise entre 3 nm et 2000 nm, et
dans laquelle la distribution du complexe métallique et du médicament dans la particule est telle que la distance moyenne entre une molécule de médicament et un complexe métallique est inférieure à 15 nm,
dans laquelle ledit ligand est de formule (I) : 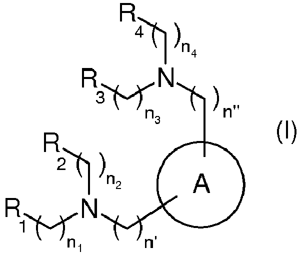 dans laquelle :
- R₁, R₂, R₃ et R₄ sont indépendamment choisis dans l'ensemble constitué par les fonctionnalités hydroxyle, carboxyle, éventuellement sous forme carboxylate, carbonyle, éther, phosphonate, phosphinate, thiolate, amino et imino,
- n₁, n₂, n₃ et n₄ sont indépendamment choisis parmi 1 à 6, et
- n' et n" sont indépendamment choisis parmi 1 à 6, et
- A est un hétérocycle saturé, partiellement saturé ou aromatique, comprenant au moins un groupe de coordination de formule -N= ou -N<, ledit hétérocycle étant éventuellement substitué par un ou plusieurs groupes lipophiles,
ou dans laquelle ledit ligand est de formule (1) : 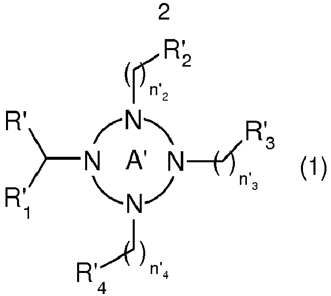 dans laquelle :
- R'₁, R'₂, R'₃ et R'₄ sont indépendamment choisis dans l'ensemble constitué par les fonctionnalités hydroxyle, carboxyle, éventuellement sous forme carboxylate, carbonyle, éther, phosphonate, phosphinate, thiolate, amino et imino,
- n'₁, n'₂ et n'₃ sont indépendamment choisis parmi 1 à 6,
- R' est choisi dans l'ensemble constitué par l'hydrogène, méthyle, les groupes alkyle en C₂ à C₂₄ linéaires ou ramifiés, et les groupes fluoroalkyle en C₁ à C₂₄, et
- A' est un cycle tétraazacycloalkyle saturé comprenant de 6 à 12 atomes de carbone.

2. Particule selon la revendication 1, dans laquelle le lanthanide est choisi dans l'ensemble constitué par Ce, Pr, Nd, Sm, Eu, Gd, Tb, Dy, Ho, Er, Tm et Yb.

3. Particule selon l'une quelconque des revendications 1 et 2, dans laquelle le médicament est choisi dans l'ensemble constitué par la doxorubicine, la mitoxantrone et l'irinotécan.

4. Particule selon l'une quelconque des revendications 1 à 3, **caractérisée en ce qu'**elle est choisie dans l'ensemble constitué par une particule coeur-coquille, un liposome, une lipoparticule, une particule solide revêtue, une particule obtenue par polymérisation, une particule gélifiée, une particule creuse, et une particule résultant d'une sonification.

5. Particule selon la revendication 4, **caractérisée en ce qu'**il s'agit d'un liposome comprenant un coeur aqueux entouré par une bicouche de phospholipide.

6. Particule selon l'une quelconque des revendications 1 à 5, pour une utilisation dans le traitement d'un cancer.

7. Particule selon l'une quelconque des revendications 1 à 5, pour une utilisation dans des méthodologies d'imagerie.

8. Composition pharmaceutique comprenant au moins une particule selon l'une quelconque des revendications 1 à 5, et au moins un excipient pharmaceutiquement acceptable.

9. Procédé pour préparer la particule selon la revendication 5, comprenant les étapes suivantes :
- préparation d'une solution comprenant un solvant organique, le complexe métallique luminescent tel que défini dans la revendication 1 et un phospholipide,
- évaporation du solvant organique afin que soit obtenu un film lipidique,
- formation de liposomes, typiquement par hydratation dudit film et sonification du mélange résultant, et
- chargement du médicament tel que défini dans la revendication 1 dans lesdits liposomes, de préférence par incubation des liposomes dans une solution dudit médicament.
